(19) Europäisches Patentamt
European Patent Office
Office européen des brevets

(11) **EP 2 719 770 B1**

(12) **EUROPEAN PATENT SPECIFICATION**

(45) Date of publication and mention
of the grant of the patent:
**30.12.2015  Bulletin 2015/53**

(51) Int Cl.:
***C12Q 1/68*** *(2006.01)*     ***G01N 21/27*** *(2006.01)*

(21) Application number: **12188542.0**

(22) Date of filing: **15.10.2012**

(54) **A method of detecting a presence and/or measuring a quantity of an analyte in a sample by a nucleic acid amplification reaction**

Verfahren zur Erkennung der Anwesenheit bzw. zur Messung der Menge eines Analyten in einer Probe mittels Nukleinsäurenamplifikationsreaktion

Procédé de détection d'une présence et/ou de mesure d'une quantité d'un analyte dans un échantillon par une réaction d'amplification d'acide nucléique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.04.2014  Bulletin 2014/16**

(73) Proprietors:
• **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**
• **Roche Diagnostics GmbH**
  **68305 Mannheim (DE)**
  Designated Contracting States:
  **DE**

(72) Inventor: **Knobel, Rolf**
**6343 Rotkreuz (CH)**

(74) Representative: **Richardt Patentanwälte PartG mbB**
**Wilhelmstraße 7**
**65185 Wiesbaden (DE)**

(56) References cited:
**WO-A2-03/029924     US-B2- 8 005 628**

• **E. B. SHAIN ET AL: "A new method for robust quantitative and qualitative analysis of real-time PCR", NUCLEIC ACIDS RESEARCH, vol. 36, no. 14, 1 January 2008 (2008-01-01), pages e91-e91, XP055055640, ISSN: 0305-1048, DOI: 10.1093/nar/gkn408**

**Description**

**Field of the invention**

The present invention relates to a method and a system of detecting a presence and measuring a quantity of an analyte in a sample by a nucleic acid amplification reaction.

**Background and prior art**

In vitro Nucleic acid amplification techniques include a variety of methods based on the two different approaches. One approach includes methods by which a DNA or RNA sequence is multiplied, thus making it more readily detectable for various procedures or tests. For example, in vitro amplification of the nucleic acid can be done using one of the following methods: Polymerase chain reaction (PCR), Ligase chain reaction (LCR), or isothermal transcription mediated amplification (TMA) method. In all of the above mentioned approaches the nucleic acid is subjected to repetitive amplification cycles. Another approach is achieving the signal amplification without subjecting the nucleic acid to the repetitive amplification cycles, as it is the case for the branched (bDNA) method.

A technique that is frequently used for various medical, biological or industrial applications is the PCR technique. This technique relies on thermal cycling, consisting of cycles of repeated heating and cooling for enzymatic replication of the nucleic acid in order to amplify a specific region of the nucleic acid strand. The PCR process comprises the following stages: (a) a baseline phase where the signal strength of the growth signal is below the measurement sensitivity (b) at the exponential amplification stage the amount of nucleic acid is doubled at every cycle; (c) at the leveling off stage the nucleic acid polymerase loses its activity because of the consumption of reagents such as nucleoside triphosphates (NTPs), primers or encymes; (d) at the plateau stage no more nucleic acid accumulates due to exhaustion of reagents and enzymes.

The PCR technique includes various methods of nucleic acid amplification. In one of these methods the quantity of nucleic acid is measured during the PCR reaction. This technique is called real-time PCR (RT-PCR) and is used to determine the presence of a nucleic acid sequence in the sample and additionally to quantify the concentration in the sample. In order to indirectly measure the amount of nucleic acid during RT-PCR various techniques are known using various fluorescent dyes, which are conjugated to the nucleic acid temporarily or permanently. This creates fluorescence signals indicating nucleic acid amplification which are measured in real time in order to provide the raw RT-PCR signal.

Though the RT-PCR technique has a high degree of precision, in order to interpret correctly the raw RT-PCR signals, additional techniques should be used to reduce or eliminate the raw RT-PCR signal variations between different replicates, runs or instruments. These techniques are called normalization methods. Raw RT-PCR signal variations can be caused by variations of reagent amount, sample concentration, sample volume, measurement temperature, or by transmission variations of the optical measurement device. The normalization of the raw RT-PCR signal is helping to reduce these variations and thus to avoid misinterpretations of the RT-PCR signal. The normalization methods are also used when needed to compare independently processed RT-PCR experiments, or in order to compare data generated by different measurement devices, or the data obtained from different sample holders or wells. Numerous normalization methods are developed in order to normalize the acquired RT-PCR signal, such as known from the following prior art documents.

US Patent No 7 788 039 B2 discloses methods, apparatus, and systems, including computer program products, implement techniques for determining an amount of target nucleic acid ('target') in a sample. Signal data is received for a plurality of cycles of an amplification experiment performed on the target and a standard nucleic acid ('standard'). The signal data includes a series of signal values indicating a quantity of standard present during cycles of the standard amplification, and a series of signal values indicating a quantity of target present during cycles of the target amplification. A target growth curve value is defined using the target signal values and a standard growth curve value is defined using the standard signal values. An initial amount of the target is calculated according to a calibration equation using an initial amount of the standard, and the target and standard growth curve values, where the calibration equation is a nonlinear equation. In columns 10 and 11 (formula 5) of this document a normalization method is described using baseline intercept division after linear baseline subtraction.

US Patent No 7 363 168 B2 mentions baseline subtraction. Particularly, the invention relates to algorithms for determining the threshold cycle for the first reliable detection of the amplified nucleic acid product.

US Patent No 8 005 628 B2 discloses systems and methods for normalizing detected emission data collected in real-time polymerase chain reaction (RT-PCR) and other reactions. In some embodiments, a sample plate can be loaded with a fluorescent dye and subjected to a real-time PCR reaction. During initial cycles, detected emissions that correspond to the background signal contributed by the plate, buffer, and other non-reactant pieces of the reaction system and chemistry can be identified. The raw emission data can be normalized by dividing the emission data by the identified baseline signal. Note: Division of the raw data by a line function mentioned her is different from division by intercept after baseline subtraction.

US 8 239 137 B2 and EP 1 798 652 B1 disclose mathematical growth curve model formulas that can be used for determining the presence of a nucleic acid in a sample.

In Shain and Clemens, "A new method of for robust quantitative and qualitative analysis of real-time PCR," Nucleic Acids Research, 2008 Vol. 36, No. 14, pages e91-e91, DOI: 10.1093/nar/gkn408, discloses a real-time method of PCR data analysis. The method calculates: a ratio at each PCR cycle that is equal to the measured real-time PCR flourencense response divided by the PCR cycle that is equal to the measured real-time PCR flourencense for the previous cycle, this quantity then has the value one subtracted from it. This transforms the roughly sigmodal shaped amplification curve to a ratio curve with a well defined peak.

## Summary of the invention

In accordance with the present invention there are provided a method and a system for detecting a presence and/or measuring a quantity of an analyte in a sample by a nucleic acid amplification reaction as claimed in the independent claims. Further, embodiments of the invention are given in dependent claims.

In accordance with embodiments of the present invention a method of detecting a presence and/or measuring a quantity of an analyte in a sample by a nucleic acid amplification reaction is provided, said method comprising: acquiring a fluorescence intensity signal of the analyte for each amplification reaction cycle of the nucleic acid amplification reaction, determining an intercept value of the signals, determining a baseline of the signals, determining a maximum growth value of the signals, normalizing the signals by modifying said signals, wherein the modification comprises calculating a difference of the signal and the baseline and dividing the difference by a divisor, wherein the divisor is proportional to a combination of the intercept and the maximum growth value, wherein the normalized signal is dimensionless, and processing the normalized signal in order to enable a detection of the presence and/or measuring the quantity of the analyte.

In accordance with the embodiments of the invention the values required for signal normalization are obtained from the acquired fluorescence intensity measurement values of the analyte by fitting a predefined curve model function to the fluorescence intensity signals. The intercept value, the baseline and the maximum growth value are then obtained from the fitted curve function that represents the growth signal.

In accordance with an embodiment of the invention processing of one or several normalized signals is performed for generating image data which is output on a display for visual inspection for a user. The user can read the display output for qualitative and/or quantitative analysis of the displayed growth signal. As an alternative or in addition the growth signal is processed for automatic qualitative and/or quantitative evaluation of the growth curve.

The term "baseline" as understood herein encompasses the portion of the growth signal where the specific signal contribution of the amplification reaction is below the reaction measurement sensitivity.

In accordance with an embodiment of the invention the cycle number Cq (quantitation Cycle number) at which the growth signal exceeds a pre-defined threshold line, e.g in relation to the baseline. This cycle number is also referred as the $C_T$ value in the art. Alternative methods for determining the Cq value from the growth signal may be used such as determining Cq by determining the maximum of the second derivation of the growth curve.

This may have the advantage that the normalized signals of different reactions show low variations at the plateau stage. Further, the normalized signal shows a low variation of the increase phases between replicates, which is important for the correct and precise calculation of Cq values and for the correct calculation of the corresponding analyte concentrations.

Further, this retains the characteristics that the normalized signals shows clear separation of the positive signal curves and negative signal curves (indicating no presence of the analyte). The negative signal curves are kept low and do not overlap with the positive signal curves. So, the negative signal curves cannot be misinterpreted as the false positive signal curves, and thus cannot lead to qualitative misinterpretation of the complete RT-PCR measurement data.

In accordance with an embodiment of the invention, the nucleic acid amplification reaction is ligase chain reaction (LCR), transcription mediated amplification (TMA), or real-time polymerase chain reaction (RT-PCR) or a similar kind of reaction.

This may have the advantage that the method described in the invention is equally applicable to various nucleic acid amplification techniques and will represent the presence/absence and quantity of any analyte created during amplification process and thus present in the sample.

In accordance with an embodiment of the invention the normalized growth signal comprises a combination of estimations for the baseline, the intercept value, and the maximum growth value. An equation can be used as a mathematical growth curve model formula for determining the growth curve from the measured fluorescence intensity values by curve fitting as it is as such known from EP 1 798 652 B1 or US 8239137 B2.

In accordance with an embodiment of the invention the signal is an RT-PCR signal, wherein the baseline is the RT-PCR signal during the initial cycles of the RT-PCR reaction, wherein the intercept value is the RT-PCR signal offset value when the RT-PCR cycle number is zero, wherein the maximum growth value is a difference between a maximum intensity of the RT-PCR signal at a plateau region and a minimum intensity of the RT-PCR signal at the baseline, wherein the plateau region is the RT-PCR signal during final cycles of the RT-PCR reaction.

In accordance with an embodiment of the invention the divisor for performing the normalization of the growth signal is calculated by multiplying the intercept value by a logarithm of a sum of one and a multiplication of first parameter by a ratio of maximum growth value and intercept value, divided by first parameter, preferably by calculating

$$yIntercept * \ln[1+ \gamma \, max.Growth / yIntercept] / \gamma,$$

wherein yIntercept is the estimation of the intercept value, $\gamma$ is the first parameter, max.Growth is the estimation of the maximum growth value.

In accordance with an embodiment of the invention the first parameter is ranging between 0.1 - 5, wherein the first parameter is preferably 0.3.

In accordance with an embodiment of the invention the divisor is calculated as the sum of the intercept value and a multiplication of the maximum growth value and a second parameter, preferably by calculating

$$yIntercept + \alpha \, max.Growth,$$

wherein yIntercept is the estimation of the intercept value, $\alpha$ is the second parameter, wherein max.Growth is the estimation of the maximum growth value.

In accordance with an embodiment of the invention the second parameter is ranging between 0.01 - 1, wherein the second parameter is preferably 0.1.

In another aspect, the invention relates to a system for detecting a presence and/or measuring a quantity of an analyte in a sample by a nucleic acid amplification reaction, said system being operable to perform an embodiment of a method of the invention.

In accordance with an embodiment of the invention the RT-PCR signal is obtained using intercalation-monitoring PCR method, TagMan (hydrolysis) PCR method, or hybridization PCR method.

In accordance with an embodiment of the invention the cycle number at which the signal exceeds the baseline is defined using a threshold value.

**Brief description of the drawings**

In the following preferred embodiments of the invention are described in greater detail by way of example only making reference to the drawings in which:

Figure 1    is a block diagram illustrating a schematic of a RT-PCR detection system,

Figure 2    illustrates schematically an un-normalized RT-PCR signal of an analyte for each RT-PCR cycle,

Figure 3    illustrates a detected un-normalized RT-PCR signal of the analyte for each RT-PCR cycle,

Figure 4    illustrates an intercept normalized RT-PCR signal of the analyte for each RT-PCR cycle,

Figure 5    illustrates a maximum growth normalized RT-PCR signal of the analyte for each RT-PCR cycle,

Figure 6    illustrates a mixed intercept maximum growth normalized RT-PCR signal of the analyte for each RT-PCR cycle,

Figure 7    illustrates determined Cq values of the mixed intercept maximum growth normalized RT-PCR signal compared to determined cycle threshold values of the intercept normalized RT-PCR signal,

Figure 8    is a flow chart illustrating a method of measuring a quantity of the analyte in a sample by RP-PCR reaction.

**Detailed description**

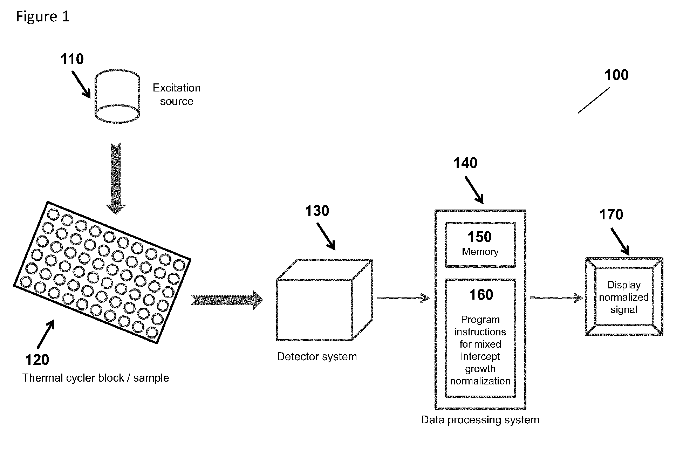

**Figure 1** shows schematics of the RT-PCR system 100 for amplification and simultaneous quantification of an analyte in real time. Using RT-PCR method, a signal is created and detected during the amplification process. The signal generally represents the amount of any analyte created during amplification and thus present in the sample. The sample is a liquid that may contain the analyte and/or other products of the amplification reaction. The RT-PCR system comprise a thermal cycler block 120, an excitation light source 110, a detector system 130 for collecting the RT-PCR signal in real time, and a data storage system 140 consisting of memory 150 for storing the RT-PCR signal and program instructions 160 for analyzing and normalizing the RT-PCR signal, and a unit 170 for displaying the signal.

The analyte is conjugated to a fluorescent dye and the sample is loaded into the thermal cycler block 120. A thermal cycler block 120 can be a conventional design sample block, which comprise 96 wells and is able to hold up to 96 samples. The sample is illuminated with the fluorescence excitation source 110, and the raw fluorescence data is measured by the RT-PCR detector system 130 for each RT-PCR cycle number. The RT-PCR detector 130 is suitable to collect the RT-PCR fluorescence signal emitted by one or more fluorescent dyes. The measured data is collected in data processing system memory unit 150, and can be displayed 170 as an un-normalized RT-PCR signal, or alternatively as a normalized RT-PCR signal.

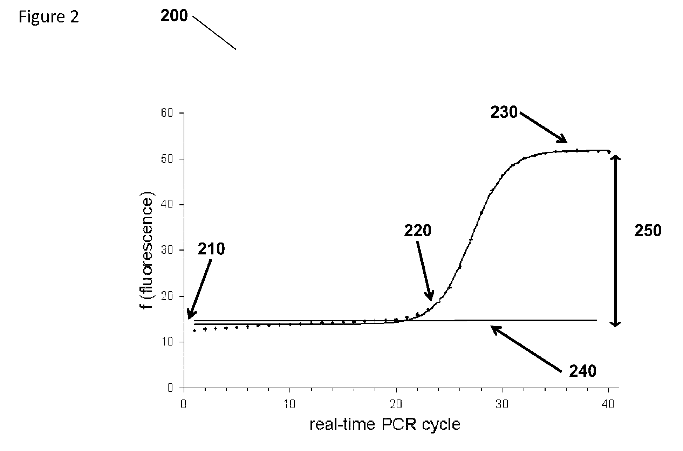

**Figure 2** shows the schematic example of a growth signal curve 200 representing the RT-PCR signal taken for each RT-PCR cycle. Figure 2 also shows fluorescence intensity values 205 as a dotted line, where each of the intensity values 205 has been acquired by performing a fluorescence intensity measurement of the ongoing amplification reaction. The intensity values 205 are modeled using a mathematical growth curve model formula or another kind of interpolation and/or interpolation. The resultant RT-PCR signal is showing as growth signal curve 200 in figure 2.

Here the intercept value 210 is the RT-PCR signal offset value when the RT-PCR cycle number is zero.

The baseline 240 is the RT-PCR signal during the initial cycles of the RT-PCR reaction, is basically the noise level in early cycles, typically measured between cycles 3 and 15, where there is no detectable increase in fluorescence due to PCR reaction products. The pre-defined hreshold line 220 is used to determine a cycle number at which the RT-PCR signal exceeds the baseline 240 of the RT-PCR reaction, i.e. the cycle in which there is the first detectable significant increase in fluorescence, which is about $C_T$ = 22 here. The maximum growth value 250 is a difference between a maximum intensity of the RT-PCR signal at a plateau region 230 and a minimum intensity of the RT-PCR signal at the baseline 240. The plateau stage 230 is the RT-PCR signal during final cycles of the RT-PCR reaction.

For example, the intercept value, baseline and maximum growth value can then be determined from coefficients from the fitted mathematical growth curve model formula, such as

$$f(x) = p_1 \cdot (1 + p_2 \cdot x) + \frac{p_3}{[1 + exp(-p_4 \cdot (x - p_5))] \cdot [1 + exp(-p_6 \cdot (x - p_7))]}$$

wherein

x represents the RT-PCR cycle number,
$p_1$ is an intercept value,
$p_2$ is a relative drift of the baseline,
$p_3$ is a distance between the baseline and the saturation line, or the maximum growth,
$p_4$ is a slope at the inflection point of a first multiplicative sigmoid function representing exponential growth,
$p_5$ is an inflection point of a first multiplicative sigmoid function,
$p_6$ is a slope at the inflection point of a second multiplicative sigmoid function, representing saturation growth,
$p_7$ is an inflection point of a second multiplicative sigmoid function, wherein optimal values of the parameters are determined by a non-linear aggression fitting algorithm to the measured data,
$p_1 * (1 + p_2 * x)$ is the baseline or a linear regression of the first intensity values 205.

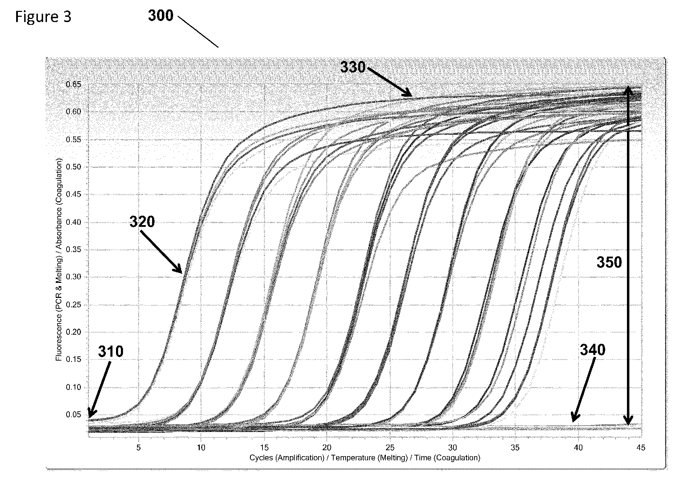

**Figure 3** shows the measurement RT-PCR signals 300 as functions of the RT-PCR cycle number. Here again the intercept value 310, growth region 320, baseline 340, maximum growth value 350 and a plateau region 330 are shown for the RT-PCR signals 300. The un-normalized RT-PCR signals 300 will be used here as a starting point in order to illustrate the result of different normalization methods, starting with prior art intercept normalization and maximum growth methods, and further proceeding to the mixed intercept growth normalization method in accordance with an embodiment of the present invention.

For accurate interpretation of RT-PCR data, the obtained raw RT-PCR signal can be adjusted for eliminating or reducing the induced RT-PCR signal variations. Signal variations can be caused by variations of sample concentration, by variations of sample volume, by change of sample temperature, or by transmission variations of particular optical measurement device. In order to compare the independently processed RT-PCR experiments, or in order to compare the data, generated by different measurement devices, or the data obtained from different sample wells, a scale invariance of acquired RT-PCR signal 300 is obtained in accordance with embodiments of the invention by normalizing the RT-PCR signal 300 . This can be reached by using ratios of measurement value estimation only, e.g. dividing the signals by a term which is proportional to a combination of intercept and growth value estimation. The correct data normalization method will lead to the scale invariance of the acquired RT-PCR signal.

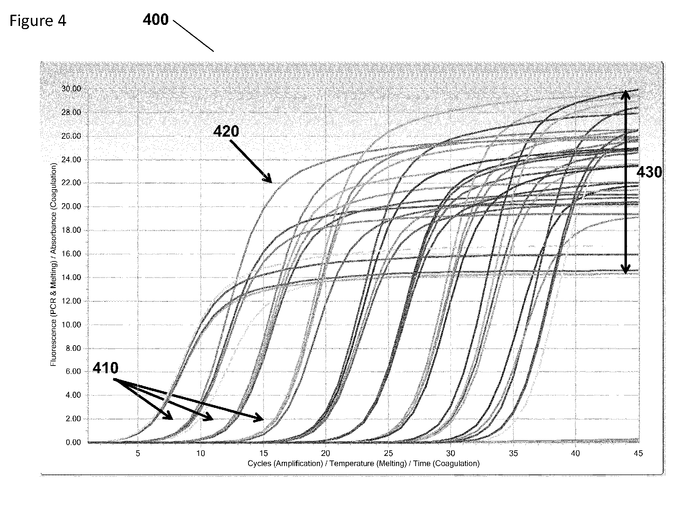

**Figure 4** shows the prior art intercept normalization method. The intercept normalized signal 400 shown on Figure 4, is calculated by dividing a difference between the RT-PCR signal 300 of Figure 3 and the baseline 340 by the corresponding intercept value 310. The intercept normalized signal 400 is preferably calculated by:

$$\text{Intercept Normalized Signal} = (\text{Signal} - \text{Baseline}) / y\text{Intercept,}$$

wherein the baseline 340 is the RT-PCR signal during the initial cycles of the RT-PCR reaction and the intercept value 310 is the RT-PCR signal offset value when the RT-PCR cycle number is zero. After intercept normalization, the baseline values of RT-PCR signal became close to zero, thus making the visual comparison of the intercept normalized signal curves easier.

In order to be able to correctly analyze the obtained normalized signal, a cycle threshold value, determined from the normalized signal graphical representation should mirror the measured threshold values with high precision. This is necessary in order to avoid misinterpretations of the RT-PCR data.

The intercept normalized RT-PCR signal shows high deviations of the signal for the replicates 410, which means high x-deviation during initial RT-PCR cycles as well as high y-variation of the signal 430 at the plateau region 420. This can cause the inprecise calculation of the cycle threshold value and lead to misinterpretation of the obtained RT-PCR signal 400.

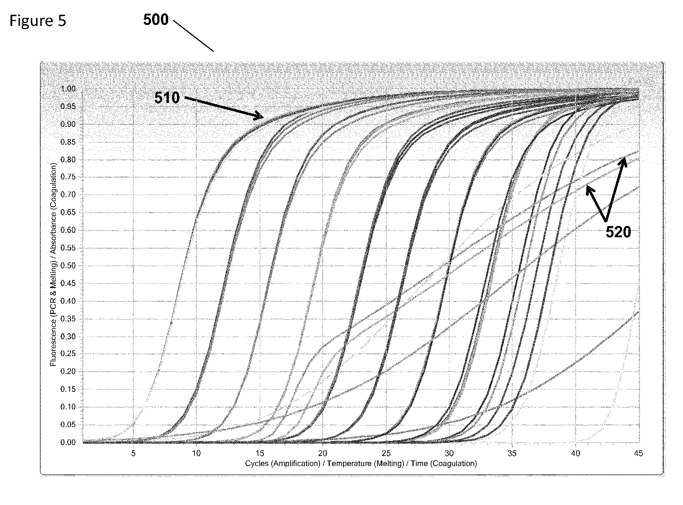

**Figure 5** shows the prior art maximum growth normalization method. The maximum growth normalized signal 500 shown on Figure 5, is calculated by dividing a difference between the RT-PCR signal of Figure 3 and the baseline 340 by the corresponding maximum growth value 350. The maximum growth normalized signal 500 is preferably calculated by:

$$\text{Growth Normalized Signal} = (\text{Signal} - \text{Baseline}) / \text{Growth,}$$

wherein the baseline 340 is the RT-PCR signal during the initial cycles of the RT-PCR reaction and the maximum growth value 350 is a difference between a maximum intensity of the RT-PCR signal at a plateau region 330 and a minimum intensity of the RT-PCR signal at the baseline 340, wherein the plateau region 330 is the RT-PCR signal during final cycles of the RT-PCR reaction.

After using the maximum growth normalization method, the normalized signal profile 500 shows that negative curves 520 are magnified higher than the positive ones 510. This fact can lead to the situation when the negative curves 520 are misinterpreted as false positive curves, further leading to qualitative misinterpretation of complete RT-PCR data.

The proposed MIG (mixed intercept growth) method according to the present invention avoids the disadvantages of the above described intercept normalization method and maximum growth normalization method, and allows determination of Cq-values with higher precision.

According to the mixed intercept maximum growth normalization method, the normalized RT-PCR signal can be calculated by dividing the difference of signal and baseline by a divisor, wherein the divisor is calculated by multiplying the intercept value by a logarithm of a sum of one and a multiplication of first parameter by a ratio of maximum growth value and intercept value, divided by first parameter, preferably by calculating:

$$\text{MIG Normalized Signal} = (\text{Signal} - \text{Background}) / (y\text{Intercept} * \ln[1 +$$
$$\gamma \, \text{max.Growth} / y\text{Intercept}] / \gamma),$$

wherein yIntercept is the estimation of the intercept value, wherein $\gamma$ is the first parameter ranging between 0.1 - 5, wherein $\gamma$ is preferably 0.3, wherein max.Growth is the estimation of the maximum growth value.

A mathematical simplification of the intercept growth normalization algorithm shows comparable results for the MIG normalized signal calculated by dividing the difference of signal and baseline by a divisor, wherein the divisor is calculated as the sum of the intercept value and a multiplication of the maximum growth value and a second parameter, preferably by calculating:

$$\text{MIG Normalized Signal*} = (\text{Signal} - \text{Background}) / (\text{yIntercept} + \alpha \text{ max.Growth}),$$

wherein yIntercept is the estimation of the intercept value, wherein $\alpha$ is the second parameter ranging between 0.01 - 1, wherein $\alpha$ is preferably 0.1, wherein max.Growth is the estimation of the maximum growth value.

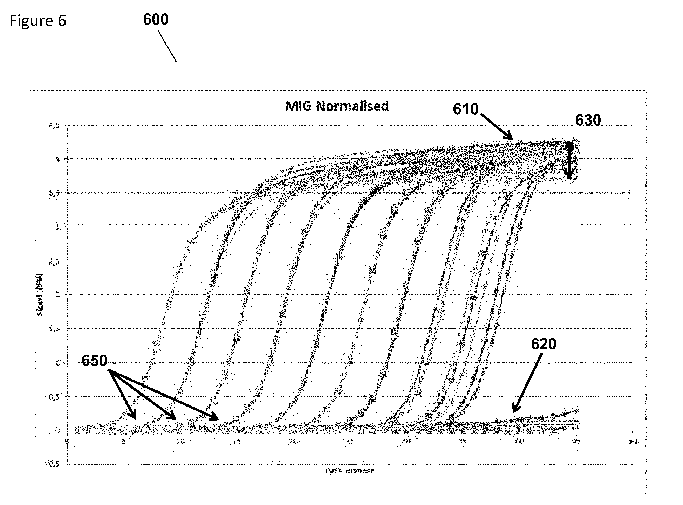

**Figure 6** shows the normalized RT-PCR signal 600 obtained by using the mathematical simplification of mixed intercept maximum growth normalization method, when the value of parameter $\alpha$ is 0.1. The normalized RT-PCR signal 600 shows clear separation of the positive curves 610 and negative curves 620. The negative curves 620 are kept low and do not interfere with the positive curves 610. So by using this type of normalization the negative curves 620 cannot be misinterpreted as the false positive curves, and thus cannot lead to misinterpretation of the complete RT-PCR data. Further, the normalized RT-PCR signal 600 shows low variations of signal amplification profiles at the plateau stage 630.

This can guarantee the accurate calculation of the cycle threshold values and thus the correct interpretation of the obtained RT-PCR signal. Further, the normalized signal 600 shows a low standard deviation of the replicates 650, which is also important for the correct calculation of cycle threshold values and for the correct calculation of correspondent analyte concentrations. Analyzing the mixed intercept maximum growth RT-PCR signal profile one can clearly conclude that the mixed intercept growth normalization does not carry the disadvantages of the prior art normalization methods (i.e. high x-deviation of the replicas signal, high y-deviations at the plateau region, or disability to separate the positive and negative curves). That is why compare to the prior art normalizations, the presented mixed intercept growth normalization is more suited to accurate RT-PCR signal processing and interpretation.

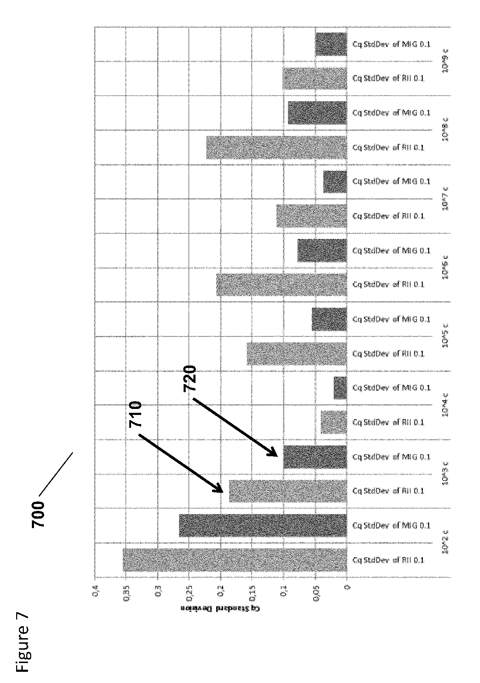

**Figure 7** shows the threshold standard deviations after the mixed intercept maximum growth normalization compared to the threshold standard deviations after the intercept growth normalization 700. The data are from replicate reactions of pooled samples and reagents in different reaction wells of one instrument. One can clearly see that the mixed intercept growth normalization leads to Cq standard deviations 720 which are significantly lower compared to the Cq standard deviations calculated using the intercept normalization 710. Low deviations of the threshold value lead to more precise interpretation of the RT-PCR data and more accurate quantitation of the analyte in the sample.

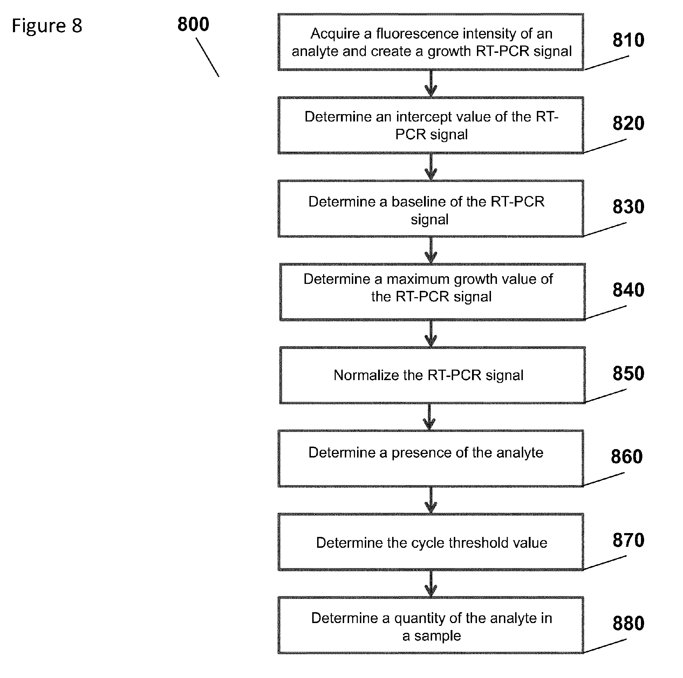

Finally, **Figure 8** shows a flow chart 800 of a method used to measure the quantity of an analyte in a sample by real-time polymerase chain reaction (RT-PCR), comprising the following steps:

- acquiring a RT-PCR fluorescence intensity of the analyte for each RT-PCR cycle and creating a growth RT-PCR signal (step 810);

- determining an intercept value of the RT-PCR signal (step 820);

- determining a baseline of the RT-PCR signal (step 830);

- determining a maximum growth value of the RT-PCR signal (step 840).

Determining of the intercept value, baseline and maximum growth value can be done by analyzing the growth signal curve, wherein the intercept value is the RT-PCR signal offset value when the RT-PCR cycle number is zero. The baseline is the RT-PCR signal during the initial cycles of the RT-PCR reaction, is basically the noise level in early cycles where there is no detectable increase in fluorescence due to PCR reaction products. The maximum growth value is a difference between a maximum intensity of the RT-PCR signal at a plateau region and a minimum intensity of the RT-PCR signal at the baseline. The plateau region is the RT-PCR signal during final cycles of the RT-PCR reaction.

Alternatively, the comprehensive mathematical kinetic function or its mathematic equivalents can be used to analytically model the measured intensity values to provide a RT-PCR growth signal curve shape, and to thus obtain the intercept value, baseline and maximum growth values as the coefficients of the mathematical kinetic function:

$$f(x) = p_1 \cdot (1 + p_2 \cdot x) + \frac{p_3}{[1 + exp(-p_4 \cdot (x - p_5))] \cdot [1 + exp(-p_6 \cdot (x - p_7))]}$$

The method further comprises normalizing the RT-PCR signal (step 850), wherein the normalization can be performed using normalization method in accordance with an embodiment of the present invention.

According to the embodiment of the present invention, the normalized RT-PCR signal can be calculated by:

$$\text{MIG Normalized Signal} = (\text{Signal} - \text{Background}) / (\text{yIntercept} * \ln[1 + \gamma \text{ max.Growth} / \text{yIntercept}] / \gamma),$$

wherein yIntercept is the estimation of the intercept value, wherein $\gamma$ is the first parameter ranging between 0.1 - 5, wherein $\gamma$ is preferably 0.3, wherein max.Growth is the estimation of the maximum growth value.

Alternatively, according to the embodiment of the present invention, the normalized RT-PCR signal can be calculated by:

$$\text{MIG Normalized Signal}^* = (\text{Signal} - \text{Background}) / (\text{yIntercept} + \alpha \text{ max.Growth}),$$

wherein yIntercept is the estimation of the intercept value, wherein $\alpha$ is the second parameter ranging between 0.01 - 1, wherein $\alpha$ is preferably 0.1, wherein max.Growth is the estimation of the maximum growth value.

The method further comprises:

- determining a presence of the analyte (step 860);
- determining the cycle threshold value of the normalized amplification signal (step 870); The threshold value is used to determine a cycle number at which the RT-PCR signal exceeds the baseline of the RT-PCR reaction, i.e. the cycle in which there is the first detectable significant increase in fluorescence, and
- determining the quantity of the analyte in the sample (step 880).

The flow chart consists of several intermediate steps, which are equally important for the interpretation of the nucleic acid amplification reaction results, and which can be displayed independently after completion of RT-PCR reaction. These intermediate steps include a qualitative discrimination step, i.e. determination of a presence or absence of the analyte; a quantitative calculation based on determining the cycle number Cq at which the normalized signal exceeds the baseline at a pre-defined threshold level; and displaying the growth curve for immediate visualization of the amplification reaction results.

Further, the mixed intercept growth normalized signal curve crosses zero and is flat by the early reaction cycles, because of the subtraction of the signal and the baseline while signal normalization, thus making the visual comparison of the normalized signal curves easier. Further, the method may have an advantage that the normalized signal is dimensionless due to normalization procedure that is made in a way that the measured signal, baseline, intercept value and maximum growth value only appear in ratios of each other, thus ensuring that the measurement signal unit cancels out in the normalization. This independence of signal scale corrects for optical and detector variances and makes comparison of curves from different wells or different instruments easier.

**List of reference numerals**

| 100 | RT PC R system |
| 110 | Excitation source |
| 120 | Thermal cycler block with a sample |
| 130 | RT-PCR detection system |
| 140 | Data processing system |
| 150 | Memory |
| 160 | Program instructions for mixed intercept maximum growth normalization |
| 170 | Display normalized signal |
| 200 | Fluorescence signal for each RT-PCR cycle |
| 205 | intensity value |
| 210 | Intercept value |
| 220 | Threshold value |
| 230 | Plateau region |

| 240 | Baseline |
|---|---|
| 250 | Maximum growth value |
| 300 | Un-normalized RT-PCR signal |
| 310 | Intercept value |
| 320 | Growth region |
| 330 | Plateau region |
| 340 | Baseline |
| 350 | Maximum growth value |
| 400 | Intercept normalized RT-PCR signal |
| 410 | High x-deviation of the RT-PCR signal for the replicates |
| 420 | Plateau region |
| 430 | High y-deviation of the RT-PCR signal at the plateau region |
| 500 | Growth normalized RT-PCR signal |
| 510 | Positive signal after using the growth normalization method |
| 520 | Negative signal after using the growth normalization method |
| 600 | Mixed intercept maximum growth normalized RT-PCR signal |
| 610 | Positive signal after using the mixed intercept growth normalization method |
| 620 | Negative signal after using the mixed intercept growth normalization method |
| 630 | Low y-deviation of the RT-PCR signal at the plateau region |
| 650 | Low x-deviation of the RT-PCR signal for the replicates |
| 700 | Comparison of the standard deviations of threshold values |
| 710 | Standard deviation of threshold values: intercept normalization |
| 720 | Standard deviation of threshold values: mixed intercept growth normalization |
| 800 | Method of measuring of quantity of an analyte in a sample by RT-PCR |
| 810 | Acquisition of the RT-PCR growth signal |
| 820 | Intercept value determination |
| 830 | Baseline determination |
| 840 | Maximum growth value determination |
| 850 | Normalization of the RT-PCR signal |
| 860 | Determination of the presence of the analyte |
| 870 | Cycle threshold value determination |
| 880 | Determination of the quantity of the analyte in the sample |

**Claims**

1. A method for analyzing a nucleic acid amplification reaction of an analyte, said method comprising:

   - acquiring a fluorescence intensity of the analyte for amplification reaction cycles of the nucleic acid amplification reaction (810) and creating from said fluorescence intensity a growth signal being indicative of a fluorescence intensity over the cycles;
   - determining an intercept value of the signal (820);
   - determining a baseline of the signal (830);
   - determining a maximum growth value of the signal (840);
   - normalizing the signal by modifying said signal (850), wherein the modification comprises calculating a difference of the signal and the baseline and dividing the difference by a divisor, wherein the divisor is proportional to a combination of the intercept and the maximum growth value, wherein the normalized signal is dimensionless;
   - processing the normalized signal in order to detect the precence/absence of an analyte (860) and/or determine the amount of an analyte (880) and/or display the normalized signal for visual quality inspection.

2. The method of claim 1, wherein the nucleic acid amplification reaction is ligase chain reaction (LCR), transcription mediated amplification (TMA), or real-time polymerase chain reaction (RT-PCR).

3. The method of claim 1 or 2, wherein normalizing the growth signal comprises calculation of a combination of the baseline, the intercept value, and the maximum growth value.

4. The method of claim 1, wherein the divisor is calculated by multiplying the intercept value (210) by a logarithm of a sum of one and a multiplication of first parameter by a ratio of maximum growth value (250) and intercept value (210), divided by first parameter, preferably by calculating **yIntercept * ln[1+ $\gamma$ max.Growth / yIntercept]** /$\gamma$,

- wherein yIntercept is the intercept value (210);
- wherein $\gamma$ is the first parameter;
- wherein max.Growth is the maximum growth value (250).

5. The method of claim 4, wherein the first parameter is ranging between 0.1 - 5, wherein the first parameter is preferably 0.3;

6. The method of claim 1, wherein the divisor is calculated as the sum of the intercept value (210) and a multiplication of the maximum growth value (250) and a second parameter, preferably by calculating **yIntercept +** $\alpha$ **max.Growth,**

- wherein yIntercept is the intercept value (210);
- wherein $\alpha$ is the second parameter;
- wherein max.Growth is the maximum growth value (250).

7. The method of claim 6, wherein the second parameter is ranging between 0.01 - 1, wherein the second parameter is preferably 0.1.

8. A system for analyzing a nucleic acid amplification reaction of an analyte, said system comprising:

- means for acquiring a fluorescence intensity of the analyte for each amplification reaction cycle of the nucleic acid amplification reaction (810) and means for creating from said fluorescence intensity a growth signal being indicative of a fluorescence intensity over the cycles;
- means for determining an intercept value of the signal (820);
- means for determining a baseline of the signal (830);
- means for determining a maximum growth value of the signal (840);
- means for normalizing the signal by modifying said signal (850), wherein the modification comprises calculating a difference of the signal and the baseline and dividing the difference by a divisor, wherein the divisor is proportional to a combination of the intercept value and the maximum growth value, wherein the normalized signal is dimensionless;
- means for processing the normalized signal in order to detect the precence/absence of an analyte (860) and/or determine the amount of an analyte (880) and/or display the normalized signals for visual quality inspection..

9. The system of claims 8, wherein the RT-PCR signal is obtained using intercalation-monitoring PCR method, TagMan (hydrolysis) PCR method, or hybridization PCR method.

10. The system of any of the previous claims 8 or 9, wherein the cycle number at which the signal exceeds the baseline is determined using a predefined criterion, such as a threshold value.

**Patentansprüche**

1. Verfahren zum Analysieren einer Nukleinsäureamplifikationsreaktion eines Analyten, wobei das Verfahren umfasst:

- Ermitteln einer Fluoreszenzintensität des Analyten für Amplifikationsreaktionszyklen der - Nukleinsäureamplifikationsreaktion (810) und Erzeugen eines Wachstumssignals, das eine Fluoreszenzintensität über den Zyklen anzeigt, aus der Fluoreszenzintensität;
- Bestimmen eines Intercept-Wertes des Signals (820);
- Bestimmen einer Grundlinie des Signals (830);
- Bestimmen eines maximalen Wachstumswerts des Signals (840);
- Normalisieren des Signals durch Modifizieren des Signals (850), wobei die Modifikation das Berechnen einer Differenz des Signals und der Grundlinie und das Teilen der Differenz durch einen Divisor beinhaltet, wobei der Divisor proportional ist zu einer Kombination des Intercept- und des maximalen Wachstumswerts, wobei das normalisierte Signal dimensionslos ist;
- Verarbeiten des normalisierten Signals, um das Vorhandensein/Fehlen eines Analyten (860) zu erfassen und/oder um die Menge eines Analyten (880) zu erfassen und/oder um das normalisierte Signal für eine visuelle Qualitätsprüfung anzuzeigen.

**2.** Verfahren nach Anspruch 1, wobei die Nukleinsäureamplifikationsreaktion eine Ligasekettenreaktion (LCR), eine transkriptionsvermittelte Amplifikation (TMA) oder eine Echtzeit-Polymerasekettenreaktion (RT-PCR) ist.

**3.** Verfahren nach Anspruch 1 oder 2, wobei das Normalisieren des Wachstumssignals das Berechnen einer Kombination der Grundlinie, des Intercept-Werts und des maximalen Wachstumswerts beinhaltet.

**4.** Verfahren nach Anspruch 1, wobei der Divisor durch Multiplizieren des Intercept-Wertes (210) mit einem Logarithmus einer Summe aus eins und einer Multiplikation eines ersten Parameters mit einem Verhältnis von maximalem Wachstumswert (250) und Intercept-Wert (210), geteilt durch den ersten Parameter, vorzugsweise durch Berechnen von $\gamma$Intercept * ln[1 + $\gamma$max.Growth / $\gamma$Intercept] / $\gamma$ berechnet wird,

- wobei $\gamma$Intercept der Intercept-Wert (210) ist;
- wobei $\gamma$ der erste Parameter ist;
- wobei max.Growth der maximale Wachstumswert (250) ist.

**5.** Verfahren nach Anspruch 4, wobei der erste Parameter zwischen 0,1 - 5 liegt, wobei der erste Parameter vorzugsweise 0,3 ist;

**6.** Verfahren nach Anspruch 1, wobei der Divisor berechnet wird als die Summe des Intercept-Wertes (210) und eine Multiplikation des maximalen Wachstumswerts (250) und eines zweiten Parameters, vorzugsweise durch Berechnen von $\gamma$Intercept + $\alpha$ max.Growth,

- wobei $\gamma$Intercept der Intercept-Wert (210) ist;
- wobei $\alpha$ der zweite Parameter ist;
- wobei max.Growth der maximale Wachstumswert (250) ist.

**7.** Verfahren nach Anspruch 6, wobei der zweite Parameter zwischen 0,01 liegt, wobei der zweite Parameter vorzugsweise 0,1 ist.

**8.** System zum Analysieren einer Nukleinsäureamplifikationsreaktion eines Analyten, wobei das System umfasst:

- eine Einrichtung zum Ermitteln einer Fluoreszenzintensität des Analyten für jeden Amplifikationsreaktionszyklen der Nukleinsäureamplifikationsreaktion (810) und eine Einrichtung zum Erzeugen eines Wachstumssignals, das eine Fluoreszenzintensität über den Zyklen anzeigt, aus der Fluoreszenzintensität;
- eine Einrichtung zum Bestimmen eines Intercept-Wertes des Signals (820);
- eine Einrichtung zum Bestimmen einer Grundlinie des Signals (830) ;
- eine Einrichtung zum Bestimmen eines maximalen Wachstumswerts des Signals (840);
- eine Einrichtung zum Normalisieren des Signals durch Modifizieren des Signals (850), wobei die Modifikation das Berechnen einer Differenz des Signals und der Grundlinie und Teilen der Differenz durch einen Divisor beinhaltet, wobei der Divisor proportional ist zu einer Kombination des Intercept-Wertes und des maximalen Wachstumswerts, wobei das normalisierte Signal dimensionslos ist;
- eine Einrichtung zum Verarbeiten des normalisierten Signals, um das Vorhandensein/Fehlen eines Analyten (860) zu erfassen und/oder um die Menge eines Analyten (880) zu erfassen und/oder um die normalisierten Signale für eine visuelle Qualitätsprüfung zu erfassen.

**9.** System nach Anspruch 8, wobei das RT-PCR-Signal unter Verwendung eines Interkalationsüberwachungs-PCR-Verfahrens, eines TagMan (Hydrolyse)-PCR-Verfahrens oder eines Hybridisierungs-PCR-Verfahrens erhalten wird.

**10.** System nach einem der vorangehenden Ansprüche 8 oder 9, wobei die Zykluszahl, bei der das Signal die Grundlinie überschreitet, unter Verwendung eines vordefinierten Kriteriums, beispielsweise eines Schwellenwerts bestimmt wird.

**Revendications**

**1.** Procédé pour analyser une réaction d'amplification d'acide nucléique d'un analyte, ledit procédé comprenant :

- l'acquisition d'une intensité de fluorescence de l'analyte pour des cycles d'une réaction d'amplification de la réaction d'amplification d'acide nucléique (810) et la création d'un signal de croissance indicateur d'une intensité de fluorescence au cours des cycles à partir de ladite intensité de fluorescence ;
- la détermination d'une valeur de l'ordonnée à l'origine du signal (820) ;
- la détermination de la ligne d'une base du signal (830) ;
- la détermination d'une valeur de croissance maximale du signal (840) ;
- la normalisation du signal par la modification dudit signal (850), où la modification comprend le calcul d'une différence entre le signal et la ligne de base et la division de la différence par un diviseur, où le diviseur est proportionnel à une combinaison des valeurs de l'ordonnée à l'origine et de la croissance maximale, où le signal normalisé est sans dimensions ;
- le traitement du signal normalisé afin de détecter la présence/l'absence d'un analyte (860) et/ou déterminer la quantité d'un analyte (880) et/ou afficher le signal normalisé pour un contrôle visuel de la qualité.

2. Procédé selon la revendication 1, dans lequel la réaction d'amplification de l'acide nucléique est une réaction en chaîne par ligase (LCR), une amplification à médiation de transcription (TMA), ou une réaction de polymérase en chaîne en temps réel (TR-PCR).

3. Procédé selon les revendications 1 ou 2, dans lequel la normalisation du signal de croissance comprend le calcul d'une combinaison de la ligne de base, de la valeur de l'ordonnée à l'origine, et de la valeur maximale de croissance.

4. Procédé selon la revendication 1, dans lequel le diviseur est calculé en multipliant la valeur de l'ordonnée à l'origine (210) par un logarithme d'une somme obtenue en ajoutant un à la multiplication d'un premier paramètre par un rapport entre une valeur maximale de croissance (250) et une valeur de l'ordonnée à l'origine (210), divisé par le premier paramètre, de manière préférable, en calculant **y ordonnée à l'origine\* ln[1 + $\gamma$ Croissance max. / y ordonnée à l'origine] / $\gamma$,**

   - où y ordonnée à l'origine est la valeur de l'ordonnée à l'origine (210) ;
   - où $\gamma$ est le premier paramètre ;
   - où Croissance max. est la valeur maximale de croissance (250).

5. Procédé selon la revendication 4, dans lequel le premier paramètre se situe dans une gamme entre 0,1 et 5, dans lequel le premier paramètre est de préférence égal à 0,3 ;

6. Procédé selon la revendication 1, dans lequel le diviseur est calculé sous la forme de la somme de la valeur de l'ordonnée à l'origine (210) et d'une multiplication de la valeur maximale de croissance (250) et d'un second paramètre, de manière préférable, en calculant **y ordonnée à l'origine + $\alpha$ Croissance max.,**

   - où y ordonnée à l'origine est la valeur de l'ordonnée à l'origine (210) ;
   - où $\alpha$ est le second paramètre ;
   - où Croissance max. est la valeur maximale de croissance (250).

7. Procédé selon la revendication 6, dans lequel le second paramètre se situe dans une gamme entre 0,01 et 1, dans lequel le second paramètre est de préférence égal à 0,1.

8. Système pour l'analyse d'une réaction d'amplification de l'acide nucléique d'un analyte, ledit système comprenant :

   - des moyens d'acquisition de l'intensité de la fluorescence de l'analyte pour chaque cycle de la réaction d'amplification de la réaction d'amplification de l'acide nucléique (810) et des moyens pour la création d'un signal de croissance étant indicateur de l'intensité de la fluorescence au cours des cycles à partir de ladite intensité de la fluorescence ;
   - des moyens de détermination d'une valeur de l'ordonnée à l'origine du signal (820) ;
   - des moyens de détermination d'une ligne de base du signal (830) ;
   - des moyens de détermination d'une valeur maximale de croissance du signal (840) :
   - des moyens de normalisation du signal par la modification dudit signal (850), où la modification comprend le calcul de la différence entre le signal et la ligne de base, et la division de la différence par un diviseur, où le diviseur est proportionnel à une combinaison de la valeur de l'ordonnée à l'origine et de la valeur maximale de croissance, où le signal normalisé est sans dimensions ;
   - des moyens de traitement du signal normalisé afin de détecter la présence/l'absence d'un analyte (860) et/ou de déterminer la quantité d'un analyte (880) et/ou d'afficher les signaux normalisés pour un contrôle visuel de la qualité.

9. Système selon la revendication 8, dans lequel le signal RT-PCR est obtenu en utilisant le protocole PCR avec suivi d'intercalation, le protocole PCR TagMan (hydrolyse), ou le protocole PCR par hybridation.

10. Système selon l'une quelconque des revendications précédentes 8 ou 9, dans lequel le numéro du cycle pour lequel le signal dépasse la ligne de base est déterminé en utilisant un critère prédéfini, tel qu'une valeur de seuil.

Figure 1

**110**

Excitation
source

**100**

**120**
Thermal cycler block / sample

**130**

Detector system

**140**

**150**
Memory

**160**

Program
instructions
for mixed
intercept
growth
normalization

Data processing system

**170**

Display
normalized
signal

Figure 2    **200**

Figure 3

Figure 4        **400**

Figure 5        **500**

Figure 6 **600**

Figure 7

Figure 8    **800**

| Acquire a fluorescence intensity of an analyte and create a growth RT-PCR signal | **810** |

↓

| Determine an intercept value of the RT-PCR signal | **820** |

↓

| Determine a baseline of the RT-PCR signal | **830** |

↓

| Determine a maximum growth value of the RT-PCR signal | **840** |

↓

| Normalize the RT-PCR signal | **850** |

↓

| Determine a presence of the analyte | **860** |

↓

| Determine the cycle threshold value | **870** |

↓

| Determine a quantity of the analyte in a sample | **880** |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7788039 B2
- US 7363168 B2
- US 8005628 B2

- US 8239137 B2
- EP 1798652 B1

**Non-patent literature cited in the description**

- **SHAIN ; CLEMENS.** A new method of for robust quantitative and qualitative analysis of real-time PCR. *Nucleic Acids Research,* 2008, vol. 36 (14), e91-e91